# EUROPÄISCHE PATENTSCHRIFT

(11) **EP 3 576 828 B1**
(45) Veröffentlichungstag und Bekanntmachung des Hinweises auf die Patenterteilung: **16.12.2020**
(21) Anmeldenummer: 18701374.3
(22) Anmeldetag: 31.01.2018
(51) Int. Cl.: A61M 25/02, A61L 29/16, A61L 29/04, A61M 39/00

(54) **KATHETERANSATZ AUS KUNSTSTOFF ENTHALTEND MOLEKULAR-DISPERS VERTEILTES POLYCHLORIERTES PHENOXYPHENOL (PCPP)**
CATHETER ATTACHMENT MADE OF PLASTIC CONTAINING MOLECULAR-DISPERSE DISTRIBUTED POLYCHLORINATED PHENOXYPHENOL (PCPP)
EMBOUT DE CATHÉTER EN PLASTIQUE COMPRENANT DU PHÉNOXYPHÉNOL POLYCHLORÉ SOUS FORME DE DISPERSION MOLÉCULAIRE (PCPP)

(30) Priorität: 31.01.2017 EP 17154003
(43) Veröffentlichungstag der Anmeldung: 11.12.2019
(73) Patentinhaber: Schierholz, Jörg Michael, 82319 Starnberg-Bete (DE)
(72) Erfinder: Schierholz, Jörg Michael, 82319 Starnberg-Bete (DE)
(74) Vertreter: dompatent von Kreisler Selting Werner - Partnerschaft von Patent- und Rechtsanwälten mbB
(86) Internationale Anmeldenummer: PCT/EP2018/052381
(87) Internationale Veröffentlichungsnummer: WO 2018/141783

(56) Entgegenhaltungen:
- WO-A1-02/066595
- WO-A2-2006/032904
- US-A1- 2003 133 831

## Beschreibung

Gegenstand der vorliegenden Erfindung ist ein Katheteransatz aus nicht-elastomeren Kunststoff enthaltend molekular-dispers verteiltes polychloriertes Phenoxyphenol (PCPP).

Infusionen durch zentrale und periphere Katheter sind elementarer Bestandteil der Intensivmedizin sowie des (perioperativen) Managements schwerkranker Patienten. Parenterale Ernährung, Flüssigkeitssubstitution, hämodynamisches Monitoring und die in der Regel kontinuierliche Medikamentengabe stellen die wichtigsten Indikationen (Schierholz, M., Rump, A. F. E., Pulverer, G., Kathetermaterialien: Schwierige Suche nach neuen Werkstoffen, Deutsches Ärzteblatt 95(17), A1007-1009, (1998)). Die Dimension der wachsenden Problematik von nosokomialen katheterassoziierten Infektionen wird anhand weniger Zahlen deutlich: Bei alleine in den USA nahezu 30 Millionen jährlich implantierten zentralen Venenkatheter (ZVK) liegt die Rate der katheterassoziierten Sepsis 2,8 bis 10% aller Intensivpatienten (D. Maki et al., The risk of bloodstream infection in adults with different intravascular devices: A systematic review of 200 published prospective studies, Mayo Clin Proc. 81(9): 1159-1171, 2006).

Die Letalitätsrate variiert je nach Literaturangabe zwischen 4-20%. Den gravierenden klinischen Konsequenzen folgen ökonomische: Verlängerung des intensivmedizinischen Aufenthaltes um bis zu 6,5 Tage, Verlängerung der Krankenhausverweildauer um insgesamt bis zu 12,5 Tage mit einem finanziellen Mehraufwand von mehr als 10.000$ (A.Simon et al, Healthcare associated infections in pediatric cancer patients: results of a prospective surveillance study from university hospitals in Germany and Switzerland, BMC Infectious disease, 8:70-79), 2008,). Kontaminierte Katheter-Ansätze, Ansatzstücke, Konnektoren, Adapter, Septen, Ansatzventile, Verbindungsstücke, Absperrhähne, Zuspritzpforten, Luer-Locks, einsteckbare oder einschraubbare Kupplungsboxen als auch Zuleitungsverschlüsse infizieren die Katheterlumina und werden nach einigen Tagen Liegedauer ebenso infektiös relevant wie der subkutane Weg (Sitges-Serra A, Linares J, Garau J: Catheter sepsis: the clue is the hub. Surgery 97 (1985) 355-357. Schierholz Jörg M, Pulverer Gerhard, Rump Alexis FE: Katheter-Materialien: Schwierige Suche nach neuen Werkstoffen. Dtsch Arztebl 95 (1998) A1006-A1009). Katheteransätze ermöglichen es, am proximalen Ende eines Katheterschlauches oder am Zuleitungs-Ventil einer peripheren Venenverweilkanüle medizinische Geräte zur Zuführung bzw. Entnahme von Flüssigkeiten mittels Injektion, Infusion oder Transfusion anzuschließen, wozu es einsteckbare oder einschraubbare Kupplungsboxen gibt. Ansätze können mit einem Verschlusselement versehen sein (Stop Cocks).

Das gleiche Problem gilt bei kontaminierten Verbindungsstücken (bzw. Spacern) einer inhalativen Therapie z.B. der medikamentösen Therapie bei COPD und Asthma, das mikrobielle Verunreinigungen hier mit in den Atmungstrakt gelangen können.

Aufgabe der vorliegenden Erfindung ist es, die oben beschriebenen Nachteile zu vermeiden und antimikrobiell aktive Katheteransätze zur Prävention von Kontaminationen und Infektionen zur Verfügung zu stellen.

Die der Erfindung zugrundeliegende Aufgabe wird durch einen antimikrobiell ausgerüsteten Katheteransatz gemäß Anspruch 1 gelöst. Die davon abhängigen Unteransprüche betreffen besondere Ausführungsformen des erfindungsgemäßen Katheteransatzes.

Im Gegensatz zu den in WO 2006/032904A2 offenbarten medizinischen Vorrichtungen ist der erfindungsgemäße Katheteransatz nicht nur mit dem erfindungsgemäß verwendeten antimikrobiellen Wirkstoff imprägniert, sondern erfindungsgemäß liegt der Wirkstoff, polychloriertes Phenoxyphenol, im nicht-elastomeren Kunststoff, aus dem der Katheteransatz besteht, molekular-dispers vor. Nur durch dieses Merkmal kann der erfindungsgemäße Katheteransatz seine vorteilhaften Eigenschaften entwickeln. Wäre der Katheteransatz nur imprägniert wie dies nach dem Stand der Technik bei elastomeren Kunststoffen möglich wäre, würde der Katheteransatz innerhalb kurzer Zeit seine antimikrobielle Wirksamkeit sehr stark einbüßen (Burst-Effekt) und seine mechanischen Eigenschaften verlieren und somit seine Aufgabe nicht mehr zuverlässig gewährleisten können. Die molekular-disperse Verteilung des antimikrobiellen Wirkstoffs ist in einem formstabilen, nicht-elastomeren Polymeren mithin gegenüber der Offenbarung gemäß WO 2006/032904A2 ein neuer und nicht naheliegender Ansatz.

WO 02/066595A1 betrifft ein Septum aus einem elastomeren Kunststoff, der zur Ausbildung eines Katheteransatzes im erfindungsgemäßen Sinne nicht geeignet ist.

Die US 2003/0133831 A offenbart Konnektoren für Katheter, bestehend aus polymerem Material (z.B. Polyurethan, Polypropylen, Polyethylen, PVC), welches mit Triclosan imprägniert ist.

Der erfindungsgemäße Katheteransatz ist aus nicht-elastomerem thermoplastischen Kunststoff enthaltend homogen molekular-dispers verteiltes polychloriertes Phenoxyphenol (PCPP), insbesondere 5-Chlor-2-(2,4-dichlorphenoxy)-phenol (Irgasan), gefertigt. Irgasan ist ein allgemein bekannter Handelsname des 5-Chlor-2-(2,4-dichlorphenoxy)-phenols.
Figur 1 zeigt Hemmhöfe von Rigidex Polyethylene + Irgasan auf *S. epidermidis* Stamm KH 6.
Figur 2 zeigt eine rasterelektronenmikroskopische Aufnahme der molekulardispersen Verteilung von 1% Rigidex-Irgasan.
Figur 3 zeigt Ergebnisse eines Fibroblasten Zytotoxizitätstest (MTT) mit verschiedenen antimikrobiellen Substanzen.

Der erfindungsgemäße Katheransatz weist homogen molekular-dispers verteiltes polychloriertes Phenoxyphenol (PCPP), insbesondere 5-Chlor-2-(2,4-dichlorphenoxy)-phenol auf. Unter dem Begriff molekular dispers verteilt versteht der Fachmann unter anderem Teilchengrößen < 1µm. Im Kontext der Erfindung versteht der Fachmann insbesondere ein im polymeren Werkstoff, aus dem der Katheteransatz gebildet ist, dispers angeordnetes polychlorierten Phenoxyphenol (PCPP), insbesondere 5-Chlor-2-(2,4-dichlorphenoxy)-phenol. Dabei ist die Größe der polychloriertes Phenoxyphenol (PCPP)-Teilchen, insbesondere 5-Chlor-2-(2,4-dichlorphenoxy)-phenol Teilchen so beschaffen, dass makroskopisch ein homogenes Material vorhanden ist. Insbesondere ist die Größe der Teilchen im polymeren Wirkstoff kleiner als 10 nm, insbesondere kleiner als 1 nm. Bei molekulardispersen Systemen handelt es sich um klare und durchsichtige Lösungen, bei denen keine Phasengrenze erkennbar ist. Sie zeichnen sich dadurch aus, dass sie physikalisch stabil und homogen sind, d.h., dass sich die gelösten Ionen und Moleküle nicht durch Filtrieren oder Zentrifugieren vom Lösungsmittel abtrennen lassen (echte Lösung). Damit unterscheiden sie sich von Substanzen, die lediglich kolloidal oder grob dispergiert, vorliegen.

Typischerweise liegt das polychlorierte Phenoxyphenol (PCPP), insbesondere 5-Chlor-2-(2,4-dichlorphenoxy)-phenol, in einer Menge von mindestens 0,01 Gew. %, insbesondere von 0,05 Gew. % bis 10 Gew. %, bezogen auf das Gewicht des Katheteransatzes im Katheteransatz vor.

Kunststoffe werden grob in Elastomere, Thermoplaste und Duroplaste eingeteilt. Die erfindungsgemäß eingesetzten Kunststoffe gehören zu den Thermoplasten.

Der Kunststoff des Katheteransatzes ist ausgewählt aus der Gruppe bestehend aus nicht-elastomeren thermoplastischen Kunststoffen. Geeignete Kunststoffe sind insbesondere Polycarbonate, nichtelastomere, harte Polyurethane, Polyethylene (bevorzugt HDPE), Polypropylene, Polybutadiene, Polybutylene, Polyketone, Polystyrole, Polysulfone, Polyethylentherephalate, Polyamide, Polyacrylate, PVC und anderen in der Medizintechnik für Katheteransätze oder Stop-Cocks verwendeten Polymeren. Nichtelastomere Kunststoffe haben beispielsweise eine Übergangstemperatur oberhalb der Raumtemperatur (25°C).. Auch nicht elastomere Kunststoffe, die eine Glasübergangstemperatur oberhalb der Temperatur der Anwendung haben, sind geeignet. Beispielsweise zeichnen sie sich durch höhere kristalline Anteile im Polymeren aus (Beispiel PE-HD ca 80% kristalliner Anteil vs. PE-LD 30%) und sind in der Regel formstabil gegenüber gebrauchsüblichen mechanischen Einflüssen. Typische Ausführungsformen des erfindungsgemäßen Katheteransatzes sind die folgenden: Konnektoren, Verbindungsstücke, Absperrhähne, Zuleitungsventile, Katheter-Ansätze, Ansatzstücke, Adaptoren, Ansatzventile, Absperrhähne, Zuspritzpforten, einsteckbare oder einschraubbare Kupplungsboxen und Luer-Locks.

Die erfindungsgemäßen Produkte sind mit molekular dispers verteilten antimikrobiellen Substanzen ausgerüstet, wobei die Kombinationen von Polymer mit PCPP mittels ähnlicher Löslichkeitsparameter (Kohäsionsenergiedichten) so austariert sind, dass die mechanischen Eigenschaften der Materialien nicht beeinflusst werden und zudem über längere Zeit eine antimikrobielle Wirkung vorhanden ist. Gleichzeitig sind Wirkstoffe und Spritzguss/Extrusionsverfahren so aufeinander abgestimmt dass die thermostabilen Wirkstoffe aus den Katheteransatzmaterialien nicht ausbluten und zudem die Produkte sterilisierbar sind und gleichzeitig keine relevante Toxizität aufweisen. Zudem sollten die PCPPs einen Schmelzpunkt unterhalb des Schmelzpunktes des thermoplastisch verarbeiteten Kunststoffes aufweisen, damit in der Schmelze eine Lösung gelingt, und gleichzeitig temperaturstabil sein. Die Erfindung stellt erstmals antimikrobielle Ansatzmaterialien für die Infusionstherapie zur Verfügung, die extrem lipophile chlorierte Bisphenole molekular-dispers im gesamten Ansatzmaterial inkorporiert haben. Im Gegensatz zu elastomeren Thermoplasten ist es bei nicht-elastomeren Thermoplasten, welche das Trägermaterial von Ansätzen sind, aufgrund der höheren Kristallinität im Kunststoff und damit geringeren Diffusionsgeschwindigkeit einer physikalisch integrierten Substanz, deutlich schwieriger, langwirksame Freisetzungssysteme zu schaffen, die eine lange antimikrobielle Wirksamkeit aufweisen. Dies gilt insbesondere für oberflächliche Beschichtungen von Ansatzmaterialien, da die Wirkstoffe in einer aufgebrachten Schicht relativ schnell eluiert sind und diese dünnen Schichten meist nicht mechanisch stabil sind.

Das Anspritzen des wirkstoffhaltigen Katheteransatzes auf einen Schlauch oder die Kunststoffbasis einer peripheren Venenverweilkanüle gelingt am besten auf anderen Kunststoffen ähnlicher Kohäsionsenergiedichte (Löslichkeitparameter), da ansonsten Rissbildungen und Entmischungen, d.h. Spalt-oder Rissbildungen an der Phasengrenze zwischen den beiden Kunststoffen möglich sind. Die molekular-disperse Verteilung des Wirkstoffes im Blend sorgt dafür, dass glatte, homogene Zwischenphasen in den thermoplastischen Verbindungsstellen zwischen dem antimikrobiellen Blend und der angespritzten Kunststoffoberfläche beispielweise eines Katheterschlauches entstehen, welche eine dauerhafte Bindung zulassen.

Gegenstand der Erfindung ist auch ein Verfahren zur Herstellung des erfindungsgemäßen Katheteransatzes aus Kunststoff, wobei Pellets des Kunststoffs mit dem Phenoxyphenol (PCPP), insbesondere 5-Chlor-2-(2,4-dichlorphenoxy)-phenol (Irgasan) innig in Kontakt gebracht werden, unter Erhalt einer Mischung, danach die Mischung aufgeschmolzen wird und der Katheteransatz durch Extrusion oder Spritzguss gefertigt wird.

Dabei wird die Mischung beispielsweise durch Überziehen der Pellets mit Phenoxyphenol (PCPP), insbesondere 5-Chlor-2-(2,4-dichlorphenoxy)-phenol (Irgasan) erhalten.

In einer anderen Ausführungsform kann die Mischung durch Vermengen der Pellets mit Phenoxyphenol (PCPP), insbesondere 5-Chlor-2-(2,4-dichlorphenoxy)-phenol (Irgasan), erhalten werden.

Ein Verfahren zur Herstellung des erfindungsgemäßen Katheteransatzes umfasst die Herstellung einer homogenen Schmelze durch vorheriges Mischen von Polymerpellets und Phenoxyphenol (PCPP), insbesondere 5-Chlor-2-(2,4-dichlor-phenoxy)-phenol (Irgasan) bis ein gleichmäßiger Überzug aus dem Antiinfektivum auf den Polymerpellets durch Erhitzen der Mischungstrommel über den Schmelzpunkt des Phenoxyphenol (PCPP), insbesondere 5-Chlor-2-(2,4-dichlorphenoxy)-phenol (Irgasan) hinaus erreicht wird. Möglich ist auch die Benetzung der Polymerpellets mit 0,1-1% eines Polyols oder Alkohols, wodurch beim späteren Zumischen das Phenoxyphenol (PCPP), insbesondere 5-Chlor-2-(2,4-dichlorphenoxy)-phenol (Irgasan) besser auf den Polymerpellets haftet und somit eine bessere Homogenität der Polymerschmelze (beispielsweise in einer Doppelschneckenvermischung) und daraufhin nach der Extrusion/Spritzguss und damit im Endprodukt, den Katheteransätzen, die gewünschte homogene, molekular-disperse Verteilung erreicht wird.

Die mit Wirkstoff beschichteten Polymerpellets können beispielsweise mit einem Babyplast Plastifizier- und Spritzaggregat (Christmann Kunststoffverarbeitung, Gummersbach-Krispe, DE) im zweistufigen Kolben-Spritzaggregat zu den Stop-Cocks (Stopfen) verarbeitet werden, wobei das Kunststoffgranulat nahezu ausschließlich durch Wärmeleitung und fast ohne Friktion verflüssigt wird.

Die DE 10 2008 033 224 A1 beschreibt einen thermoplastischen Kunststoff mit porösen metallischen Silberagglomeraten.

Das Gebrauchsmuster G 93 20 337 U beschreibt die Integration von Silberchlorid in Ansatzmaterialien, die G 91 00 743 U beschreibt die Inkorporierung von Silberchlorid in eine Zuspritzpforte einer peripheren Venenverweilkanüle.

Die DE 33 02 567 A1 offenbart die oberflächliche Beschichtung einer Röhre mit einem Metall.

Die US 5 069 907 beschreibt die Inkorporierung von Triclosan in einen Film chirurgischer Abdeckmaterialien aus flexiblen Polyethylen. Hierbei werden elastomere Thermoplasten, vorzugsweise elastisches PE, in einem Blow-Film-ExtrusionsVerfahren oder in einem flüssigen Acryl-Harz zu flexiblen Abdeckfolien mit einer Klebeschicht verarbeitet.

Die US 2007/0299409 A1 offenbart die Integration unterschiedlicher antimikrobieller Substanzen in bio-resorbierbare Polymere.

Die WO 86/02006 beschreibt das Coating von Katheteransätzen mit unterschiedlich antimkrobiellen Substanzen.

Das US Patent 5,856,005 beschreibt die Herstellung eines antimikrobiellen antientflammbaren Garns mit chlorierten Phenoxy- Substanzen, vorzugsweise in Zellulose-acetat.

Die EP 2956510 A1 betrifft den Einbau von antimikrobiellen Substanzen in Polymerbeschichtungen, welche mit UV Licht gehärtet werden (entspricht der PCT/US 2014/015614).

Die US Patentschrift 6,106,505 offenbart die Imprägnierung einer synergistischen Kombination von Triclosan und Chlorhexidin mittels Eintauch-Techniken in Elastomere.

Das US Patent 8,277,826 B2 betrifft eine Methode, um unterschiedliche antimkrobielle Substanzen in Harze zu mischen, mit einem spezifischen Verhältnis von hydrophilen zu hydrophoben Monomer-Anteilen des Harzes.

Es ist empfehlenswert das richtige Polymer mit der antimikrobiellen Substanz 5-Chloro-2-(2,4-dichlorophenoxy)-phenol zu kombinieren, um ein langandauerndes mikrobiell aktives System herzustellen. Bei Kunststoffen wie ABS (Acrylonitrilebutadien Styrene) Polymeren wurden zu geringe Freisetzungsraten erzielt, da beide Komponenten nicht kompatibel waren (Effects of triclosan incorporation into ABS plastic on biofilm communities, Journal of Antimicrobial Chemotherapy (2004) 53, 989-996, DOI: 10,1093/jacidkh196).

Überraschenderweise konnten mit dem bevorzugten antimikrobiellen Mittel 5-Chloro-2-(2,4-dichlorophenoxy)phenol mit den erfindungsgemäß ausgewählten Polymeren im Spritzguss- und Extrusionsverfahren homogene Polymerblends mit langandauernder Wirksamkeit entwickelt werden zur Herstellung der erfindungsgemäßen Katheteransätze. 5-Chloro2-(2,4-dichlorophenoxy)phenol ist eine Substanz welche in vielen Produkten verwendet wird von Seifen, Deodorants und Spielzeug bis zur Dekolonisierung der Patienten von MRSA mit einer 2%igen wässrigen Lösung (A. D. Russell, Whither triclosan? Journal of Antimicrobial Chemotherapy (2004) 53, 693-695, DOI: 10.1093/jac/dkh171). Die Aktivität ist bei höheren Konzentrationen bakterizid mit multiplen Targets im Zytoplasma und an der Zellmembran, bei niedrigeren Konzentrationen bakteriostatisch, welches grundsätzlich für den Gebrauch von antimikrobiellen Verbindungsstücken bei guter Bioverfügbarkeit ausreicht. Die Inkorporierung wird so gewählt, dass einerseits ausreichende antimikrobielle Wirkung über eine längere Zeitgewährleistet ist und andererseits keine relevanten Toxizitäten oder das Ausbluten des Produktes entstehen.

Die Erfindung wird anhand der vorliegenden Beispiele näher erläutert.

### Beispiel 1

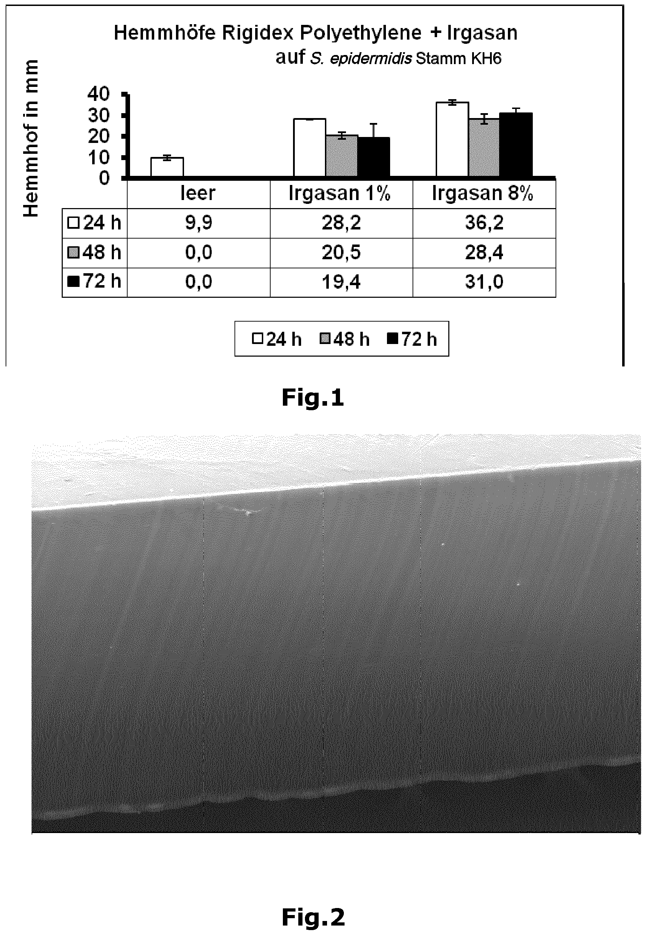

Polyethylengranulat (Rigidex) wurde in einem Mischer mit 1% und 8% Irgasan versehen und in einem Minispritzgussgerät (Babyplast Plastifizier- und Spritzaggregat (Christmann, Gummersbach-Krispe, DE) zu kleinen 2mm dicken Scheiben verblendet. Die antibakteriellen Hemmhöfe wurden nach 24, 48 und 72h sowie nach 144h gegen *S. epidermidis* Stamm KH6 getestet. Nach 48h bildete sich ein stabiles Plateau bei einem ca. 20mm (1%), 28-30mm (8%) Hemmhof aus bis zu einem getesteten Zeitpunkt von 144h (Figur 1).

### Beispiel 2

Polycarbonat (Bayer AG) wurde in einem Mischer mit 1% und 8% Irgasan versehen und in einem Minispritzgussgerät zu kleinen 2mm dicken Scheiben verblendet. Die Hemmhöfe wurden nach 24, 48 und 72h sowie nach 144h getestet. Nach 48h bildete sich ein stabiles Plateau bei einem ca. 18mm (1%), bzw. 38mm (8%) Hemmhof aus bis zu einem getesteten Zeitpunkt von 144h.

### Beispiel 3

Polyamid wurde in einem Mischer mit 1% und 8% Irgasan versehen und in einem Minispritzgussgerät zu kleinen 2mm dicken Scheiben verblendet. Die Hemmhöfe wurden nach 24, 48 und 72h sowie nach 144h getestet. Nach 48h bildete sich ein stabiles Plateau bei einem ca. 22 mm (1%), bzw. 39 mm (8%) Hemmhof aus bis zu einem getesteten Zeitpunkt von 144h.

### Beispiel 4

Bakterienadhäsion: 1% Polyamid-Irgasanl% Polyamid-Irgasan wurden mit einer Übernachtkultur *S. epidermidis* Stamm KH11 bei ca. 10-4 CFU in MHB (Müller-Hinton Bouillon) 1h inkubiert und dann in MHB 24 h bebrütet. Daraufhin wurde in einem Abdrucktest die Anzahl adhärierender CFU bestimmt. Es wurde keine Kolonien gezählt.

### Beispiel 5

REM Bilder für molekular-disperse Verteilung 1% Rigidex-Irgasan (Fig. 2).

### Beispiel 6

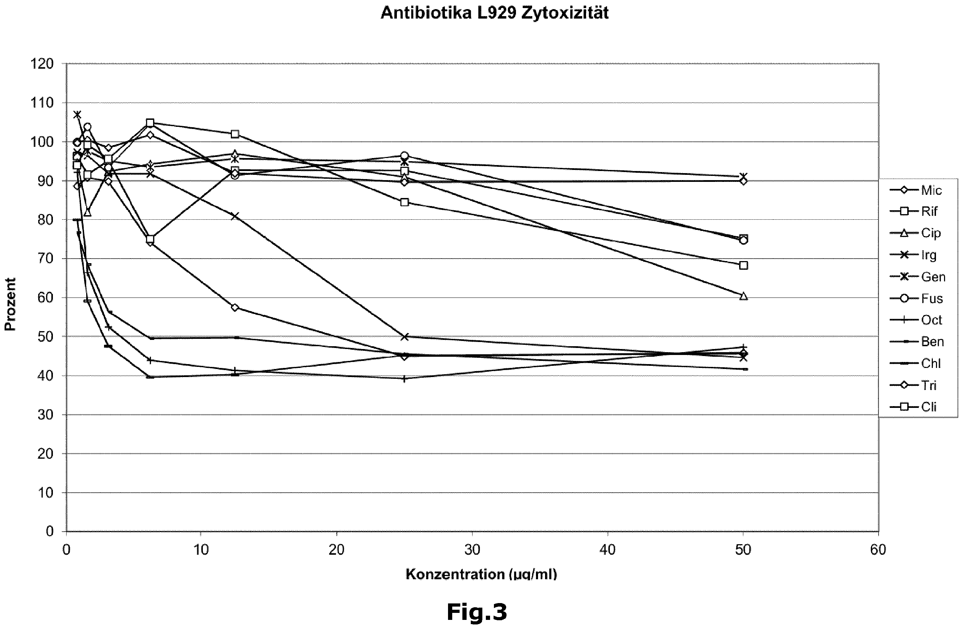

Die Freisetzungsraten für Hemmhöfe von ca. 20 mm wurden so angesetzt, dass bei 2-4mg/I Irgasan Freisetzung(cm²) eine nur minimale Beeinträchtigung beim Fibroblasten-Zytotox-Test gefunden wurde (Fig. 3). In Fig. 3 bedeuten Mic: Miconazol, Tri : Trimetoprim, Rif: Rifampicin, Irg: Irgasan, Gen: Gentamicin, Fus: Fusidinsäure, Oct: Octenidin, Ben: Benzylpenicillin, Chl: Chlorhexidin, Cli: Clindamycin. Fig. 3 zeigt, dass bis zu einer Konzentration von maximal 10 µg/ml die Fibroblasten kaum gehemmt werden und die Substanz untoxisch ist - im Gegensatz zu den Antiseptika Chlorhexidin und Octenidin.

## Patentansprüche

1. Katheteransatz aus nicht-elastomerem thermoplastischen Kunststoff enthaltend homogen molekular-dispers verteiltes polychloriertes Phenoxyphenol (PCPP), insbesondere 5-Chlor-2-(2,4-dichlorphenoxy)-phenol (Irgasan).

2. Katheteransatz nach Anspruch 1, wobei das polychlorierte Phenoxyphenole (PCPP), insbesondere 5-Chlor-2-(2,4-dichlorphenoxy)-phenol, in einer Menge von mindestens 0,05 Gew. % bezogen auf das Gewicht des Katheteransatzes vorliegt.

3. Katheransatz gemäß Anspruch 2, wobei das polychlorierte Phenoxyphenol (PCPP), insbesondere 5-Chlor-2-(2,4-dichlorphenoxy)-phenol, in einem Bereich von 0,05 Gew. % bis 10 Gew. % bezogen auf das Gewicht des Katheteransatzes vorliegt.

4. Katheteransatz nach einem der Ansprüche 1 bis 3, wobei der Kunststoff des Katheteransatzes ausgewählt ist aus der Gruppe bestehend aus Polycarbonaten, nicht-elastomeren, harten Polyurethanen, Polyethylen, Polypropylen, Polybutadien, Polybutylen, Polyketon, Polystyrol, Polysulfon, Polyethylentherephalat, Polyamiden, Polyacrylate, PVC und anderen in der Medizintechnik verwendeten Polymeren.

5. Katheteransatz nach einem der Ansprüche 1 bis 4, wobei der Katheteransatz ausgewählt ist aus der Gruppe bestehend aus Konnektoren, Verbindungsstücke, Absperrhähne, Zuleitungsventile, Katheter-Ansätze, Ansatzstücke, Adaptoren, Ansatzventile, Absperrhähne, Zuspritzpforten, einsteckbare oder einschraubbare Kupplungsboxen, Spacer und Luer-Locks.

6. Verfahren zur Herstellung eines Katheteransatzes nach einem der Ansprüche 1 bis 5 aus Kunststoff, wobei Pellets des Kunststoffs mit dem Phenoxyphenol (PCPP), insbesondere 5-Chlor-2-(2,4-dichlorphenoxy)-phenol (Irgasan) innig in Kontakt gebracht werden, unter Erhalt einer Mischung, danach die Mischung aufgeschmolzen wird und der Katheteransatz durch Extrusion oder Spritzguss gefertigt wird.

7. Verfahren nach Anspruch 6, wobei die Mischung erhältlich ist durch Überziehen der Pellets mit Phenoxyphenol (PCPP), insbesondere 5-Chlor-2-(2,4-dichlorphenoxy)-phenol (Irgasan).

8. Verfahren nach Anspruch 6, wobei die Mischung erhältlich ist durch Vermengen der Pellets mit Phenoxyphenol (PCPP), insbesondere 5-Chlor-2-(2,4-dichlorphenoxy)-phenol (Irgasan).

## Claims

1. A catheter hub made of a non-elastomeric thermoplastic material comprising homogeneously molecularly dispersed polychlorinated phenoxyphenol (PCPP), especially 5-chloro-2-(2,4-dichlorophenoxy)phenol (Irgasan).

2. The catheter hub according to claim 1, wherein said polychlorinated phenoxyphenol (PCPP), especially 5-chloro-2-(2,4-dichlorophenoxy)phenol, is present in an amount of at least 0,05% by weight, based on the weight of the catheter hub.

3. The catheter hub according to claim 2, wherein said polychlorinated phenoxyphenol (PCPP), especially 5-chloro-2-(2,4-dichlorophenoxy)phenol, is present in a range of from 0.05% by weight to 10% by weight, based on the weight of the catheter hub.

4. The catheter hub according to any of claims 1 to 3, wherein the plastic of the catheter hub is selected from the group consisting of polycarbonates, non-elastomeric rigid polyurethanes, polyethylene, polypropylene, polybutadiene, polybutylene, polyketone, polystyrene, polysulfone, poly(ethylene terephthalates), polyamides, polyacrylates, PVC, and other polymers used in medical technology.

5. The catheter hub according to any of claims 1 to 4, wherein said catheter hub is selected from the group consisting of connectors, connecting pieces, stopcocks, feed line valves, catheter hubs, extensions, adapters, hub valves, stopcocks, injection ports, insertable or screwable coupling boxes, spacers, and Luer locks.

6. A process for preparing a catheter hub according to any of claims 1 to 5 made of plastic, in which pellets of the plastic are brought into intimate contact with the phenoxyphenol (PCPP), especially 5-chloro-2-(2,4-dichlorophenoxy)phenol (Irgasan), to obtain a mixture, followed by melting the mixture and preparing the catheter hub by extrusion or injection molding.

7. The process according to claim 6, wherein said mixture is obtainable by coating the pellets with phenoxyphenol (PCPP), especially 5-chloro-2-(2,4-dichlorophenoxy)phenol (Irgasan).

8. The process according to claim 6, wherein said mixture is obtainable by admixing the pellets with phenoxyphenol (PCPP), especially 5-chloro-2-(2,4-dichlorophenoxy)phenol (Irgasan).

## Revendications

1. Embout de cathéter en plastique thermoplastique non-élastomère contenant du polychlorophénoxyphénol (PCPP) réparti de manière homogène en dispersion moléculaire, en particulier du 5-chloro-2-(2,4-dichlorophénoxy)phénol (Irgasan).

2. Embout de cathéter selon la revendication 1, dans lequel le polychlorophénoxyphénol (PCPP), en particulier le 5-chloro-2-(2,4-dichlorophénoxy)phénol, est présent dans une quantité d'au moins 0,05 % (m/m) par rapport à la masse de l'embout de cathéter.

3. Embout de cathéter selon la revendication 2, dans lequel le polychlorophénoxyphénol (PCPP), en particulier le 5-chloro-2-(2,4-dichlorophénoxy)phénol, est présent dans un intervalle de 0,05 % (m/m) à 10 % (m/m) par rapport à la masse de l'embout de cathéter.

4. Embout de cathéter selon l'une des revendications 1 à 3, dans lequel le plastique de l'embout de cathéter est sélectionné parmi le groupe constitué des polycarbonates, des polyuréthanes rigides non-élastomères, du polyéthylène, du polypropylène, du polybutadiène, du polybutylène, des polycétones, du polystyrène, des polysulfones, du poly(téréphtalate d'éthylène), des polyamides, des polyacrylates, du PVC et d'autres polymères utilisés en technique médicale.

5. Embout de cathéter selon l'une des revendications 1 à 4, dans lequel l'embout de cathéter est sélectionné parmi le groupe constitué des connecteurs, des pièces de jonction, des robinets d'arrêts, des vannes d'alimentation, des embouts de cathéter, des pièces d'embout, des adaptateurs, des vannes d'embout, des ports d'injection, des boîtiers de couplage à enficher ou à visser, des espaceurs et des raccords luer-lock.

6. Procédé de production d'un embout de cathéter selon l'une des revendications 1 à 5 en plastique, dans lequel des granulés du plastique sont amenés en contact intime avec le phénoxyphénol (PCPP), en particulier le 5-chloro-2-(2,4-dichlorophénoxy)phénol (Irgasan) pour obtenir une combinaison, la combinaison est ensuite fondue et l'embout de cathéter est fabriqué par extrusion ou moulage par injection.

7. Procédé selon la revendication 6, dans lequel la combinaison peut être obtenue par enrobage des granulés avec du phénoxyphénol (PCPP), en particulier du 5-chloro-2-(2,4-dichlorophénoxy)phénol (Irgasan).

8. Procédé selon la revendication 6, dans lequel la combinaison peut être obtenue par mélange des granulés avec du phénoxyphénol (PCPP), en particulier du 5-chloro-2-(2,4-dichlorophénoxy)phénol (Irgasan).
